(19) Europäisches Patentamt
European Patent Office
Office européen des brevets

(11) Publication number: **0 258 191 B1**

(12) **EUROPEAN PATENT SPECIFICATION**

(45) Date of publication of patent specification: **27.05.92**

(51) Int. Cl.[5]: **C07D 473/06**, A61K 31/52

(21) Application number: **87810481.9**

(22) Date of filing: **25.08.87**

(54) **Xanthine derivatives.**

(30) Priority: **28.08.86 GB 8620825**
**30.01.87 GB 8702129**
**13.02.87 GB 8703435**

(43) Date of publication of application:
**02.03.88 Bulletin 88/09**

(45) Publication of the grant of the patent:
**27.05.92 Bulletin 92/22**

(84) Designated Contracting States:
**AT BE CH DE ES FR GB GR IT LI LU NL SE**

(56) References cited:
**AT-B- 212 847**
**DE-B- 1 024 968**
**FR-A- 2 157 727**

**JOURNAL OF MEDICINAL CHEMISTRY, vol. 29, no. 6, June 1986; D.J. Blythin et al.: "Antiinflammatory activity of substituted 6-hydroxyprymido-[2,1-f]purine-2,4,8(1H,3H,9-H)-triones. Atypical nonsteroidal antiinflammatory agents", pages 1099-1113**

(73) Proprietor: **SANDOZ AG**
**Lichtstrasse 35**
**CH-4002 Basel(CH)**
(84) Designated Contracting States:
**BE CH ES FR GB GR IT LI LU NL SE**

(73) Proprietor: **SANDOZ-PATENT-GMBH**
**Humboldtstrasse 3**
**W-7850 Lörrach(DE)**
(84) Designated Contracting States:
**DE**

(73) Proprietor: **SANDOZ-ERFINDUNGEN Verwaltungsgesellschaft m.b.H.**
**Brunner Strasse 59**
**A-1235 Wien(AT)**
(84) Designated Contracting States:
**AT**

(72) Inventor: **Kis, Zoltan L.**
**Höhenweg 22**
**CH-4102 Binningen(CH)**
Inventor: **Morley, John**
**Lilienstrasse 64**
**CH-4123 Allschwil(CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

The present invention relates to novel xanthine derivatives having pharmaceutical utility, processes for their production, pharmaceutical compositions comprising them and their use as pharmaceuticals.

More particularly the present invention provides: a compound of formula I

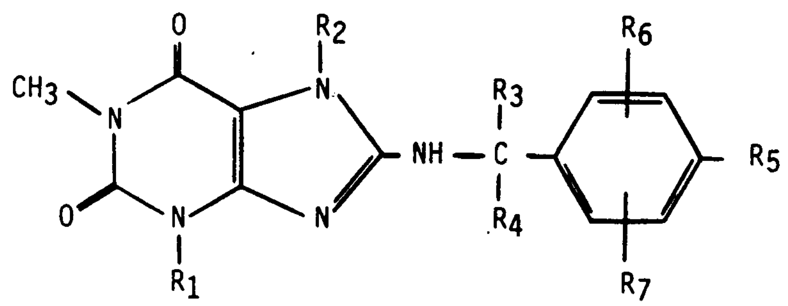

wherein

$R_1$ is $C_{1-4}$alkyl, $C_{3-4}$alkenyl or ($C_{3-5}$cycloalkyl)-methyl,

$R_2$ is $C_{1-4}$alkyl, [hydroxy- or ($C_{1-4}$alkoxy)-substituted $C_{1-3}$alkyl]-methyl, ($C_{3-5}$cycloalkyl)-methyl or a group of formula A

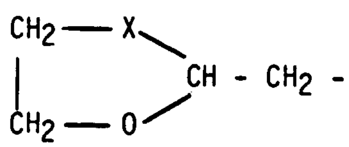 (A)

in which X is $-CH_2-$ or -O-,

$R_3$ and $R_4$ are each hydrogen, hydroxymethyl, methoxymethyl or N,N-dimethylcarbamoyloxymethyl,

$R_5$ is hydroxy or methoxy,

$R_6$ is hydrogen, hydroxy, methoxy or halogen and

$R_7$ is in the 2- or 3-position and is hydroxy, methoxy or halogen, or together with $R_5$ is 3,4-methylenedioxy, or together with $R_6$ is 2,3-methylenedioxy,

or physiologically-hydrolysable and -acceptable ester thereof.

Alkyl and alkenyl groups as $R_1$, as well as alkyl groups and the alkoxy and/or alkyl moieties of (hydroxy- and alkoxy-alkyl)-methyl groups as $R_2$, may each be branched or straight chain. [Hydroxy- and alkoxy-substituted alkyl]-methyl groups as $R_2$ may be mono-, di- or poly-substituted. Preferably they are monosubstituted. Preferred [hydroxy- and alkoxy-substituted alkyl]-methyl groups as $R_2$ are thus ($C_{1-3}$hydroxyalkyl)-methyl and ($C_{1-4}$alkoxy-$C_{1-3}$alkyl)-methyl.

When $R_1$ is alkenyl, the double bond thereof is preferably separated from the nitrogen atom to which it is attached by at least two carbon atoms. When $R_1$ is cycloalkylmethyl, this is suitably cyclopropylmethyl.

When $R_2$ is a group of formula A this is tetrahydrofuran-2-yl-methyl or 1,3-dioxolan-2-yl-methyl.

By halogen is meant fluorine, chlorine or bromine, especially fluorine or chlorine and, most especially fluorine.

In the compounds of formula I, $R_1$ is suitably methyl. The followwing significances for $R_2$ to $R_7$ inclusive are preferred, individually, collectively or in any combination or sub-combination:

1. $R_2$ is $C_{1-4}$alkyl, ($C_{1-3}$hydroxyalkyl)-methyl, ($C_{1-4}$alkoxy-$C_{1-3}$alkyl)-methyl, ($C_{3-5}$cycloalkyl)-methyl or a group of formula A. When $R_2$ is ($C_{1-3}$hydroxyalkyl)-methyl this is preferably 2-hydroxyethyl. When $R_2$ is ($C_{1-4}$alkoxy$C_{1-3}$alkyl)-methyl this is preferably 2-($C_{1-2}$alkoxy)ethyl, e.g. 2-methoxyethyl.

2. $R_3$ is hydrogen and
$R_4$ is hydrogen, hydroxymethyl, methoxymethyl or N,N-dimethyl-carbamoyloxymethyl, especially hydroxymethyl or methoxy-methyl.

3. $R_5$ is methoxy.

4a. $R_6$ is hydrogen, hydroxy or methoxy, especially hydrogen.

4b. When $R_6$ is other than hydrogen:
$R_6$ is preferably in the 5-position.

5a. $R_7$ is hydroxy or methoxy, especially methoxy.

5b. $R_7$ is in the 3-position.

In one embodiment the present invention provides:

a compound of formula I as illustrated above wherein

| | |
|---|---|
| $R_1$ | is $C_{1-4}$alkyl, $C_{3-4}$alkenyl or cyclopropylmethyl, |
| $R_2$ | is $C_{1-4}$alkyl, [hydroxy- or ($C_{1-4}$alkoxy)-substituted $C_{1-3}$alkyl]-methyl or a group of formula A as defined above, |
| $-C(R_3)R_4-$ | is $-CH_2-$, $-CH(CH_2OH)-$, $-CH(CH_2OCH_3)$, $-C(CH_2OH)_2-$ or $-C(CH_2OCH_3)_2-$, and |
| $R_5$, $R_6$ and $R_7$ | have the meanings given for formula I, or physiologically-hydrolysable and -acceptable ester thereof. |

By the term "physiologically-hydrolysable and -acceptable ester is meant an ester which is hydrolysable under physiological conditions to yield an acid which is itself physiologically acceptable, i.e. which exhibits no undesirable side effects at desired dosage levels. Such esters may be derived e.g. by acylation of free hydroxy groups in the substituents $R_2$, $R_3$ and $R_4$. Appropriate esters include e.g. those with both mono- and dicarboxylic acids having 2 to 4 carbon atoms, for example esters of compounds of formula I in which $R_2$ is acetoxyethyl and/or $R_4$ is acetoxymethyl, as in the case of the compounds of examples 12, 13 and 16 hereinafter.

Compounds of formula I and esters thereof wherein $R_3$ and $R_4$ are different exist in both S- and R-isomeric form. Similarly compounds of formula I and esters thereof wherein $R_1$ and/or $R_2$ include one or more asymmetric carbon atoms also exhibit optical isomerism. The present invention is to be understood as embracing both individual isomeric forms is well as mixtures, e.g. racaemic and diastereomeric mixtures, thereof unless otherwise specified.

Where the compounds of the invention exist in isomeric form as aforesaid, individual isomers may be obtained in conventional manner, e.g. employing optically active starting materials, e.g. as hereinafter described for examples 22 to 25 or by separation of initially obtained mixtures, e.g. racemic mixtures. In so far as utility in accordance with the present invention resides, in the case of compounds of formula I wherein $-C(R_3)R_4-$ is $-CH(CH_2OH)-$, $-CH(CH_2OCH_3)-$ or $-CH[CH_2O-CO-N(CH_3)_2]-$ and esters thereof, primarily in those compounds and esters wherein the grouping $-C(R_3)R_4-$ has the R-configuration, the R-isomers in pure or substantially pure form, or mixtures, e.g. racemic mixtures, comprising the R-isomer will be preferred.

Accordingly in a specific aspect the present invention provides:

a compound of formula I as illustrated above wherein $R_1$, $R_2$, $R_5$, $R_6$ and $R_7$ have the meanings hereinbefore given and $-C(R_3)R_4-$ is (R) $-CH(CH_2OH)-$, (R) $-CH(CH_2OCH_3)-$ or (R) $-CH[CH_2O-CO-N(CH_3)_2]-$, or a physiologically- hydrolysable and -acceptable ester thereof.

In addition to the foregoing the present invention also provides:

a process for the production of compounds of formula I and esters thereof as defined above, which process comprises:

a) for the production of a compound of formual I having a free hydroxy group, deprotecting a hydroxy-protected derivative thereof;

b) for the production of a compound of formula I, reacting a compound of formula II

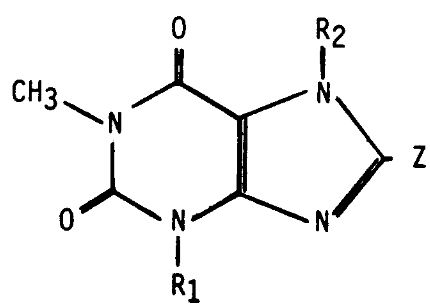

(II)

wherein $R_1$ and $R_2$ have the meanings given above and Z is a leaving group, with a compound of formula III

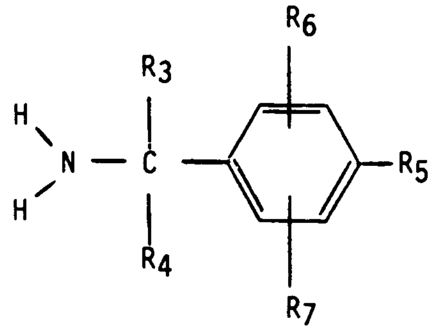

(III)

wherein $R_3$ to $R_7$ have the meanings given above, whereby any hydroxy group present in the compound of formula II or III may be in free or protected form and, when required, carrying out process step a),

c) for the production of a physiologically-hydrolysable and -acceptable ester of a compound of formula I, esterifying a compound of formula I having a free hydroxy group, any hydroxy group present which is not to be esterified being, when required, in protected form, employing an appropriate acid or derivative thereof and, when required, carrying out process step a); and, if desired,

d) separating optically active isomers from any mixture of such isomers obtained in accordance with steps a), b) or c).

Process step a) may be carried out in accordance with means known in the art for the removal of hydroxy protecting groups, e.g. for the deprotection of benzyl-protected hydroxy groups, by ether cleavage, for example via hydrogenation in the presence of a Pd/charcoal catalyst.

Process step b) above can be carried out in accordance with means generally known in the art, for example by reaction of a compound of formula II with a compound of formula III at a temperature of from ca. 20 to 180 °C in the presence of an inert solvent or diluent, suitably in the presence of an acid binding agent such as triethylamine. Suitable leaving groups as Z include e.g. halogen atoms, in particular chlorine or bromine atoms.

Process step c) is also conventional and may be performed e.g. by esterification employing an appropriate acid halide or acid anhydride, suitably in the presence of an acid binding agent such as pyridine, e.g. at a temperature of from 20 to 100 °C.

Where hydroxy groups are present in the starting materials of formula I, II or III which might otherwise be susceptible to undesired side reaction, these may be in protected form. Suitable hydroxy-protecting groups include any of those known and commonly employed in the art including e.g. benzyl. Such protecting groups are the removed subsequent to the main defined reaction in accordance with process step a).

Use of optically active starting materials for process steps a) through c) will lead directly to optically active compounds of formula I or esters thereof. Alternatively, individual optically active isomers may be recovered from initially obtained mixtures, e.g. racemic or diastereomeric mixtures, thereof in accordance with process step d) and employing any of the techniques known and commonly practiced in the art.

The starting materials of formula II and III are known or may be produced analogously to known techniques, in the case of compounds of formula II for example, by 7N-alkylation of theophylline, or analogues thereof wherein the substituent at the 4-position is other than methyl, so as to introduce the desired group $R_2$.

The following examples are illustrative of the processes of the present invention:

Example 1:

8-[α-hydroxymethyl-(3,4-dimethoxy-benzylamino)]-caffeine [formula I: $R_1 = R_2 = CH_3$; $R_3 = H$; $R_4 = HOCH_2$-; $R_5 = CH_3O$-; $R_6 = H$; $R_7 =$ (3) $CH_3O$-].

5 g 8-chlorocaffeine and 5.6 g α-hydroxymethyl-3,4-dimethoxybenzylamine are dissolved, together with 8 ml triethylamine in 200 ml ethanol. The reaction mixture is heated in an oil bath at 170 °C, with stirring, in an autoclave for 76 hours, evaporated and the residue crystallised from ethanol. The pure title compound is obtained following further washing with water: m.p. = 217 - 218 °C.

The following compounds of formula Ia may be obtained analogously:

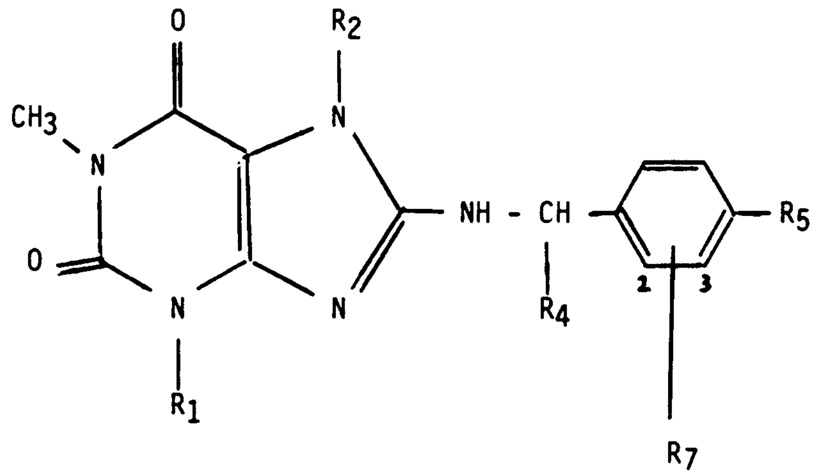

| EXAMPLE | $R_1$ | $R_2$ | $R_4$ | $R_5$ | $R_7$ | m.p.(°C) |
|---|---|---|---|---|---|---|
| 2 | $CH_3$- | $CH_3$- | H | $CH_3O$- | (3)$CH_3O$- | 237-239 |
| 3 | $CH_3$- | $CH_3O-(CH_2)_2$- | H | $CH_3O$- | (2)$CH_3O$- | 147-149 |
| 4 | $CH_2$=CH-$CH_2$- | $CH_3O-(CH_2)_2$- | H | $CH_3O$- | (3)$CH_3O$- | 116-117 |
| 5 | $CH_2$=CH-$CH_2$- | HO-$(CH_2)_2$- | H | $CH_3O$- | (3)$CH_3O$- | 163-164 |
| 6 | $CH_3$- | $CH_3O-(CH_2)_2$- | H | $CH_3O$- | (3)$CH_3O$- | 154-154.5 |
| 7 | $CH_3$- | $CH_3O-(CH_2)_2$- | HO-$CH_2$- | $CH_3O$- | (3)$CH_3O$- | 139-140 |
| 8 | $CH_3$- | HO-$(CH_2)_2$- | HO-$CH_2$- | $CH_3O$- | (3)$CH_3O$- | 192-193 |
| 9 | $CH_3$- | HO-$(CH_2)_2$- | H | $CH_3O$- | (3)$CH_3O$- | 174-175 |
| 10 | 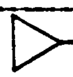 $CH_2$- | $CH_3O-(CH_2)_2$- | H | $CH_3O$- | (3)$CH_3O$- | 130-131 |
| 11 | $CH_3$- | $CH_3O-(CH_2)_2$- | $CH_3O$-$CH_2$- | $CH_3O$- | (3)$CH_3O$- | 106-109 |
| 12 | $CH_3$- | $CH_3O-(CH_2)_2$- | $CH_3$-CO-O-$CH_2$- | $CH_3O$- | (3)$CH_3O$- | 140.5-141 |
| 13 | $CH_2$=CH-$CH_2$- | 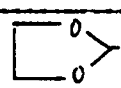 $CH_2$- | $CH_3$-CO-O-$CH_2$- | $CH_3O$- | (3)$CH_3O$- | 113-114 |
| 14 | $CH_2$=CH-$CH_2$- | 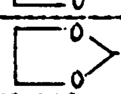 $CH_2$- | H | $CH_3O$- | (3)$CH_3O$- | 126-128 |
| 15 | $CH_3$- | 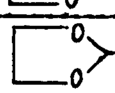 $CH_2$- | H | $CH_3O$- | (3)$CH_3O$- | 200-201 |
| 16 | $CH_3$- | $CH_3$-CO-O-$(CH_2)_2$- | H | $CH_3O$- | (3)$CH_3O$- | 162.5-163 |

| EXAMPLE | $R_1$ | $R_2$ | $R_4$ | $R_5$ | $R_7$ | m.p.(°C) |
|---|---|---|---|---|---|---|
| 17 | 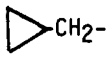 | 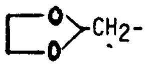 | H | $CH_3O-$ | (3)$CH_3O-$ | 139.5-141 |
| 18 | $CH_2=CH-CH_2-$ | 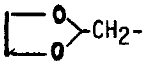 | $HOCH_2-$ | $CH_3O-$ | (3)$CH_3O-$ | 113-115 |
| 19 | $CH_3-$ | $HO-(CH_2)_2-$ | $CH_3O-CH_2-$ | $CH_3O-$ | (3)$CH_3O-$ | 147-149 |
| 20 | $CH_3-$ | 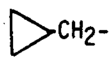 | $HOCH_2-$ | $CH_3O-$ | (3)$CH_3O-$ | ① |
| 21 | $CH_3-$ | $CH_3O-(CH_2)_2-$ | $(CH_3)_2N-CO-O-CH_2-$ | $CH_3O$ | (3)$CH_3O-$ | ② |

The compounds of examples 12, 13 and 16 above are prepared by acetylation of the compounds of examples 7, 18 and 9 respectively employing acetic acid anhydride. The reaction is performed in conventional manner in the presence of pyridine and $CH_2Cl_2$ as solvent at a temperature of ca. 20 °C.

The compound of example 19 is prepared by debenzylation of the corresponding compound in which $R_2$ is in O-benzyl protected form, this being in turn produced anlogously to example 1 employing 7-benzyloxy-ethyl-bromotheophylline in place of 8-chlorocaffeine. Debenzylation is effected by hydrogenation at 1 atm, and 20 °C in ethanol emplyoing a 10 % Pd/C catalyst.

① m.p. for the R-isomer = 165 - 166 °C, $[a]_D^{20}$ = +30° (c=1,1 in $CH_3OH$).

② m.p. for the R-isomer = 54 - 56 °C, $[a]_D^{20}$ = +23° (c=1.1 in $CH_2Cl_2$).

The R-isomer product of example 20 is obtained employing R-(α-hydroxymethyl-3,4-dimethoxy benzyl)-amine as starting material hereinafter described for example 22. The R-isomer product of example 21 is obtained from the product of example 22 hereinafter by reaction with dimethylcarbonylchloride in dioxane as solvent in the presence of sodium hydride at a temperature of ca. 20 °C.

The following compounds of formula Ib are prepared analogously to example 1, in the case of the compounds of examples 22 and 23, employing the indicated optically active starting materials, in the case of the compounds of examples 24 and 25, by phase-transfer alkylation of the products of examples 22 and 23 as described.

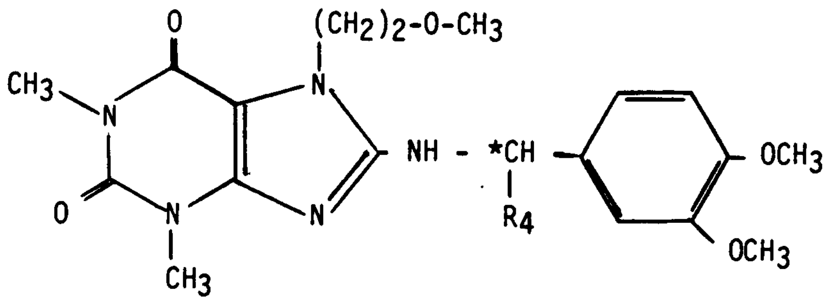

(Ib)

| EXAMPLE | $R_4$ | CONFIGURATION AT * | m.p. (°C) | $[\alpha]_D^{20}$(c = in $CH_2Cl_2$) |
|---|---|---|---|---|
| 22 | $HOCH_2$- | R | 129-130 | + 15.3 (1) |
| 23 | $HOCH_2$- | S | 129-130 | - 16.0 (1 |
| 24 | $CH_3O$-$CH_2$- | R | 104-105 | + 55.7 (1.05) |
| 25 | $CH_3O$-$CH_2$- | S | 104-105 | - 56.5 (1.01) |

Starting materials for examples 22 and 23

8-Bromo-(2-methoxyethyl)-theophylline with:
22. R-(α-hydroxymethyl-3,4-dimethoxybenzyl)amine,
23. S-(α-hydroxymethyl-3,4-dimethoxybenzyl)amine.

Preparation of the products of examples 24 and 25

5 g NaOH is dissolved in 10 ml water and supplemented at anbient temperature with 0.7 ml benzyltrimethylammonium hydroxide. 1.1 g of product compound from example 22 or 23 is dissolved in 15 ml dichloromethane to provide the second phase. The two phases are mixed with vigorous stirring and 1 ml dimethylsulfate is added drop-wise over 8 hrs. Stirring is continued for a further 20 hrs., the resulting mixture diluted with dichloromethane, separated and the organic phase extracted with diluted tartaric acid, washed with water and dried over $Na_2SO_4$. The obtained product is then purified chromatographically employing silica gel with ethylacetate / 1 % methanol as eluant.

In relation to the present invention the isomers of examples 22 and 24 above are preferred to the isomers of examples 23 and 25 respectively as exhibiting a higher level of activity as evidenced in animal models a hereinafter described.

Compounds of formula I and their esters as hereinbefore defined posess bronchodilator and anti-asthmatic activity as may be shown in standard test models, e.g. as follows:

EXAMPLE A. BRONCHODILATOR ACTIVITY

1. Bronchospasmolytic activity in vitro

1.1. Isolated guinea-pig trachea:

The trachea is excised from freshly sacrificed guinea-pigs and transected in the transverse plane to give rings of tissue of ca. 2 mm. Individual rings are mounted vertically on stainless steel supports, one of which is fixed at the base of an organ bath, the other being attached to a tension transducer. The rings are bathed in modified Tyrode solution at 37°C and gassed with $O_2/CO_2$ (95:5, v/v). Rings prepared in this manner, preloaded with 1 g, generate spontaneous tone and, after a period of equilibration, relax on addition of spasmolytic drugs. Tension can be enhanced by addition of carbachol ($10^{-6}$ M) or histamine ($10^{-4}$ M). To ascertain spasmolytic activity, test substances are dissolved in physiological saline and added in increasing quantities to the organ bath at 5 min. intervals to provide a cumulative concentration-effect curve.

In the above test model compounds and esters of the invention produce concentration-related relaxation of guinea-pig tracheal ring preparations irrespective of the contractile agency at concentrations of from about $5 \times 10^{-7}$ to about $10^{-5}$ M with respect to basal tone, from about $1{,}5 \times 10^{-5}$ to about $10^{-4}$ M in the presence of carbachol, and from about $10^{-7}$ to about $10^{-5}$ in the presence of histamine.

1.2. Isolated human bronchus:

Rings of bronchus (ca. 2mm depth) are dissected from samples of human lung, resected from patients with lung carcinoma but grossly free of disease. Activity is determined employing the methodology of example A.1.1.

In the above test model compounds and esters of the invention produce contraction-related relaxation of human bronchus ring preparations irrespective of the contractile agency at dosages of from about $10^{-6}$ to about $10^{-4}$ M.

2. Bronchodilator activity in vivo

2.1. Inhibition of bronchospasm:

Guinea pigs are anaesthetised with pentobarbital (30 mg/kg i.p.) and phenobarbital (100 mg/kg i.p.) and ventilated via a tracheal cannula (10 ml/kg, IHz). Ventilation is monitored either by a pressure transducer measuring air-flow (Konzett-Rossler method), or by a Fleisch flow transducer in line with the inspiratory circuit. When making measurements of flow, coincident pressure changes in the thorax are monitored directly via an intrathoracic trochar, permitting display of differential pressure relative to the trachea. From this information resistance and compliance are calculated at each inspiration.

Intravenous injection of bombesin (ca. 500 µg/kg) as a bolus, induces increased airways resistance which is sustained over a period of several minutes. Capacity of test-substance to reverse response when administered i.v. at the height of bombesin-induced bronchospasm serves as a measure of efficacy in reversing established bronchospasm.

In the above test model, compounds and esters of the invention are found to effect dose related abrogation of bronchospasm at dosages of from about 0.01 to about 1.0 mg/kg i.v.

2.2. Inhibition of bronchoconstriction following pulmonary administration:

Conscious guinea-pigs are subjected to inhalation of test substance or placebo (vehicle) 10 mins. prior to explosure to a 0.3% mist of acetylcholine. Test substance is administered as a mist generated from aerosol preparations at concentrations of from 1 mg/ml to 0.001 mg/ml. Prolongation of time prior to collapse in treated groups as compared with placebo groups is taken as a measure of bronchodilator efficacy.

In the above test model, detectable protection against bronchospasm is evident employing compounds and esters of the invention on introduction into the exposure chamber in 1ml amounts at the above indicated concentrations.

EXAMPLE B: SUPPRESSION OF AIRWAYS HYPERREACTIVITY

1. Sensitised Animals

Guinea pigs are anaesthetised and airways resistance and compliance recorded as described under example A.2.1. above. Intravenous injection of histamine (1 - 1.8 µg/kg) is used to define airways sensitivity. Allergic reaction is initiated by i.v. injection of preformed immune complexes (bovine γ-globulin/anti-bovine γ-globulin), using a dose that is scarcely sufficient to induce bronchospasm at the first injection. This dose

of immune complexes is repeated at regular (10 min.) intervals.

Following the last dose of immune complexes, the dose-effect relationship to histamine is re-defined. In animals so treated, induction of airways hyperreactivity is consistently observed.

On advance administration of compounds and esters of the invention at dosages of from about 0.03 to about 3.0 mg/kg i.v., suppression of induced airways resistance is observed as compared with untreated controls.

2 PAF-Treated Animals

Guinea pigs are anaesthetised and prepared for recording of lung function as described under example A.2.1. above. Intravenous injection of histamine establishes airways sensitivity to spasmogens. Following infusion of PAF (platelet activating factor) over 1hr. (total dose = 600 ng/kg), repeated injection of histamine reveals development of airways hyperreactivity, which can conveniently be expressed as the paired difference between the response amplitude before and after PAF exposure.

On administration of compounds and esters of the invention by infusion during PAF exposure at dosages of from aout 0.1 to about 20 mg/kg i.v., suppression of airways hyperreactivity induction is observed.

EXAMPLE C: INFLUENCE ON EOSINOPHIL ACCUMULATION

Effect of test-substance is conveniently determined by measurement of influence on PAF-induced eosinophil accumulation in the guinea-pig peritoneal cavity in vivo. In the guinea pig, there is a substantial (up to 40%) resident population of eosinophils in the peritoneal cavity and eosinophil accumulation in the peritoneal cavity relative to control animals ca. 24 - 48 hrs. following injection of PAF i.p. at dosages of ca. 10µg/kg serves to establish the influence of test substance on eosinophil accumulation.

To establish eosinophil accumulation, test animals receive 10µg/kg PAF i.p., 2 days prior to sacrifice. Smears from the peritoneal cavity are prepared employing Leishman's Stain after fixation with 95% methanol. At least 500 white cells are differentiated for each estimation. Test substance is administered s.c. via a minipump for four days prior to sacrifice.

On administration of compounds and esters of the invention in the above test model at dosage rates of from about 0.1 to about 10.0 mg/kg/day s.c., for a number of days prior to sacrifice and employing non-PAF-treated animals, decrease in resident eosinophil population may be observed as compared with untreated controls.

Having regard to their bronchospasmolytic activity as evidenced in test methods as described in example A above, compounds and esters of the invention are indicated for use as bronchodilators, e.g. for the treatment, e.g. symptomatic treatment of obstructive or inflammatory airways disease, for example asthma, pneumoconiosis or bronchitis. Having regard to their activity a) in inhibiting acute response in hypersensitive subjects following allergen or other challenge elliciting hypersensitivity reaction (e.g. following induction of hyperreactivity and airways obstruction via PAF challenge), b) in suppressing development of airways hyperreactivity subsequent to challenge as under a), and c) in diminishing basal, or on-going, airways hyperreactivity, as evidenced in test methods as described in example B above, compounds and esters of the invention are indicated for use in the prophylactic treatment of obstructive or inflammatory airways disease, for example the prophylactic treatment of pneumoconiosis and, in particular, asthma.

[For further discussion of the relevance of a), b) and c) above and their relationship to prophylactic utility in treating inflammatory airways disease, see e.g.: Altounyan, Clin. Allergy (supp.) 10, 481-489 (1980), Morley et al., Lancet ii, 1142-1144 (1984), Mazzoni et al., J. Physiol., 365, 107 P (1985), Traietti et al., Respiration, 46 62-63 (1984); Taytard et al., Am. Rev. Repiratory Disease, 134, 983-985 (1986); Szezeklik et al., Thrombosis and Hematosis, 56, 283-287 (1986); Basran et al., Clin Allergy, 14, 75-79 (1984), Karlsson et al., Brit. J. Clin. Pharmacol. 27, 371-374 (1985), and Mazzoni et al., Brit. J. Pharmacol., 86, 571P (1985)].

As bronchodilator agents, compounds and esters of the invention may be used to abort or restrict bronchoconstrictor attack consequential to obstructive or inflammatory airways disease, e.g. asthma (symptomatic treatment). As prophylactic agents, compounds and esters of the invention may, by continued administration, be used to provide advance protection against recurrence of bronchoconstrictor attack consequential to obstructive or inflammatory airways disease, e.g. asthma, or for the control, restriction or reversal of basal status of such disease, e.g. for the control, restriction or reversal of basal causes of asthma and asthma attack. The words "treatment" and "treating" as used throughout the present specification and claims are accordingly to be understood as including prophylactic as well as symptomatic modes, unless otherwise specified.

In accordance with the foregoing the present invention accordingly also provides:

I. A method for the treatment of obstructive or inflammatory airways disease in a subject in need thereof, which method comprises administering to said subject an effective amount of a compound of formula I as hereinbefore defined or a physiologically-hydrolysable and -acceptable ester thereof for example;

Ia. A method of effecting bronchodilatation in a subject in need thereof (for example a subject exhibiting obstructive or inflammatory airways disease or airways obstruction, including chronic or acute obstruction, for example as occurring in the symptomatology of diseases, disorders or conditions as herein set forth), which method comprises administering to said subject a bronchodilatorily effective amount of a compound of formula I as hereinbefore defined or a physiologically-hydrolysable and -acceptable ester thereof or

Ib. A method for the prophylactic treatment of obstructive or, more particularly, inflammatory airways disease (e.g. for advance protective treatment against acute airways obstruction, for example bronchospasm, e.g. as occurring in the symptomatology of diseases, disorders or conditions as herein set forth) in a subject in need thereof, which method comprises administering to said subject a prophylactically effective amount of a compound of formula I as hereinbefore defined or a physiologically-hydrolysable and -acceptable ester thereof.

In the alternative the present invention provides:

II. A compound of formula I as hereinbefore defined or a physiologically-hydrolysable and -acceptable ester thereof for use as a pharmaceutical, for example for use in the treatment of obstructive or inflammatory airways disease, e.g. for use in a method as defined under I, Ia or Ib above.

The method of the present invention as defined under I to Ib above is, in particular, applicable to the treatment of asthma of whatever type or genesis. It is applicable to both intrinsic and, especially, extrinsic asthma. It is especially applicable to the treatment of allergic asthma, whether atopic, (i.e. IgE-mediated) or non-atopic, as well as e.g. bronchitic asthma, thymic asthma, excercise induced asthma, occupational asthma, asthma induced following bacterial infection and other non-allergic asthmas. Treatment of asthma is also to be understood as embracing treatment of subjects, e.g. of less than 4 or 5 years of age, exhibiting wheezing symptoms, in particular at night, and diagnosed or diagnosable as "wheezy infants", an established patient category of major medical concern and now more correctly identified as incipient or early-phase asthmatics. (For convenience of definition this particular asthmatic condition is referred to hereinafter as "wheezy-infant syndrome").

In a series of particular embodiments the present invention thus provides for treatment of asthma, in particular allergic asthma (for example allergic atopic asthma), exercise induced asthma and wheezy-infant syndrome, including symptomatic treatment of asthma (e.g. bronchodilator treatment of asthma exacerbation or attack) as well as prophylactic treatment of asthma (e.g. prophylactic treatment of asthma exacerbation or attack), comprising use of or administration of a compound of formula I as hereinbefore defined or a physiologically-hydrolysable and -acceptable ester thereof.

The method of the present invention as defined under I to Ib above is also applicable to the treatment of pneumoconiosis (an inflammatory, commonly occupational, disease of the lungs, frequently accompanied by airways obstruction, whether chronic or acute, and occasioned by repeated inhalation of dusts) of whatever type or genesis, including, for example, aluminosis, anthracosis, asbestosis, chalicosis, ptilosis, siderosis, silicosis, tabacosis and, in particular, byssinosis.

In a further series of particular embodiments the present invention thus also provides for the treatment of pneumoconiosis, in particular byssinosis, including symptomatic treatment of airways obstruction (e.g. bronchodilator treatment of acute or chronic airways obstruction, e.g. dyspnea or bronchospasm) attributable thereto, as well as prophylactic treatment of airways obstruction (e.g. advance protective treatment of acute airways obstruction, e.g. bronchospasm) attributable thereto, comprising use or administration of a compound of formula I as hereinbefore defined or a physiologically-hydrolysable and -acceptable ester thereof.

The method of the present invention as defined under I or, especially, Ia above, is also applicable to the treatment of bronchitis or, more especially, the treatment of chronic or acute airways obstruction, for example, dyspnea, associated therewith. In this respect the present invention is applicable to the treatment of bronchitis of whatever type or genesis, including, for example, acute bronchitis, arachidic bronchitis, catarrhal bronchitis, chronic bronchitis, croupous bronchitis, phthinoid bronchitis and so forth.

In a further series of particular embodiments the present invention accordingly provides for the treatment of bronchitis or, more especially, the symptomatic treatment of airways obstruction (e.g. bronchodilator treatment of acute or chronic airways obstruction, e.g. dyspnea) attributable thereto, comprising use or administration of a compound of formula I as hereinbefore defined or a physiologically-hydrolysable and -acceptable ester thereof.

Having regard to activity of compounds and esters of the invention in suppressing eosinophil accumulation as may be demonstrated in test models such as described in example C above the present invention also provides:

III A method for the suppression of eosinophil accumulation and/or activation, e.g. for the treatment of disease characterised by or having an aetiology comprising morbid eosinophil accumulation and/or activation, in a subject in need of such treatment which method comprises administering to said subject an effective amount of a compound of formula I as hereinbefore defined or a physiologically-hydrolysable and -acceptable ester thereof;

or, in the alternative:

IV A compound of formula I as hereinbefore defined or a physiologically-hydrolysable and -acceptable ester thereof for use in a method as defined under III above.

Diseases as defined under III above include, in particular, hypereosinophilia and the eosinophil related disorders.

Hypereosinophilia is a distinct condition or status of varied aetiology characterised by chronic, morbid eosinophil presence in the body tissues generally. The eosinophil-related disorders comprise a distinct and extensively documented indication group, commonly occurring concomitant to another, primary disease or condition. [For more detailed discussion see e.g.: Schatz et al., Medical Clinics of North America, 65, (5), 1055-1071 (1981) and Ottesen et al., "Allergy, Principles and Practice", Eds.E. Middleton, C. Reed and S. Ellis, 584-632, (1987)]. The group includes eosinophil-related disorders of the airways (involving morbid eosinophilic infiltration of pulmonary tissues) as well as of other organs and tissues including, for example, the skin, eye, nasal passages and the gastro-intestinal and urinary tracts.

Eosinopil-related disorders to which the present invention is applicable include those concomitant to atopy or atopic reactions in general (e.g. atopic conditions such as rhinitis, conjunctivitis etc... as set forth below) as well as non-atopic eosinophil-related disorders.

Disorders of the airways to which the present invention is applicable include hypereosinophilia as well as, for example, eosinophil-related disorders of the airways consequential or concomitant to Löffler's syndrome, eosinophilic pneumonia, parasitic (in particular metazoan) infestation (including tropical eosinophilia), bronchopulmonary aspergillosis, polyarteritis nodosa (including Churg-Strauss syndrome) as well as eosinophil-related disorders affecting the airways occasioned by drug-reaction.

Other eosinophil-related disorders to which the present invention is applicable include eosinophilia consequential or concomitant to eosinophilic gastroenteritis, Heiner's syndrome, atopic dermatitis, urticaria or angioderma (allergic, recurrent or prolonged), ichthyosis, exfoliative dermatitis or pityriasisrubra, urticaria pigmentosa or mastocytoma, toxic epidermal necrolysis (drug related), dermatitis herpetiformis, allergic rhinitis, hyperplastic sinusitis, interstitial nephritis (drug related), interstitial cystitis, choleostatic hepatotoxicity (drug related), allergic conjunctivitis, vernal conjunctivitis, eosinophilic fascitis, hypersensitivity angiitis, serous myocarditis or endomyocardial fibrosis, Wiscott-Aldrich syndrome, selective IgA deficiency with atopy, eosinophilic leukemia and eosinophilic granuloma.

As will be appreciated, the present invention is directed primarily to the treatment of hypereosinophilia or eosinophil-related disorders as such. Where, however, eosinophil-related disorders are concomitant to atopy, for example to any of the atopic diseases or conditions specifically recited above including atopic or allergic forms of dermatitis, urticaria, angioderma, rhinitis, conjunctivitis and gastro-intestinal allergies, the present invention may equally be applicable to the treatment of eosinophil-related disorder as an integral or basal component thereof. The present invention thus also provides means for the treatment (e.g. symptomatic or prophylactic treatment) of atopy, including each of the said recited atopic diseases or conditions, as such. In treating eosinophil-related disorders concomitant to non-atopic diseases or conditions on the other hand, the compound and esters of the invention will more commonly be administered together with other medication for treatment of the disease or condition with which eosinophilia is associated. Thus in the treatment of eosinophilia consequential to parasitic infection, use will generally be in conjunction with other, anti-parasitic drug therapy.

Where compounds and esters of the invention are employed in accordance with the method of the invention for the treatment of eosinophil-related disorders of the airways, e.g. for the treatment of hypereosinophilia as it affects the lungs or for the treatment of pulmonary eosinophilia associated with eosinophilic pneumonia, and the disorder is accompanied by symptoms of airways obstruction, they may be employed either as symptomatic or prophylactic therapy, e.g. either to alleviate or abort, or to provide advance protection against recurrence of, obstruction. More commonly however compounds and esters of the invention will be employed symptomatically, e.g. as a means for the treatment of hypereosinophilia or eosinophil-related disorder, i.e. in accordance with methods defined under III above.

In a further series of particular embodiments the present invention thus also provides:

i) for the treatment of hypereosinophilia and of eosinophil-related disorders, including treatment in accordance with the methods defined under III above, including, in the case of eosinophil-related disorders of the airways associated with airways obstruction, symptomatic treatment of airways obstruction (e.g. bronchodilator treatment of acute or chronic airways obstruction, e.g. dyspnea or bronchospasm) and prophylactic treatment of airways obstruction (e.g. advance protective treatment of acute airways obstruction, e.g. bronchospasm) attributable thereto, comprising use or administration of a compound of formula I as hereinbefore defined or a physiologically-hydrolysable and -acceptable ester thereof; as well as

ii) for the treatment of atopy, for example for the treatment of any of the atopic diseases or conditions causal to or associated with eosinophil-related disorder as hereinbefore set forth, comprising use or administration of a compound of formula I as hereinbefore defined or a physiologically-hydrolysable and -acceptable ester thereof.

For the above mentioned uses an indicated daily dosage for oral administration in particular for the symptomatic and/or prophylactic treatment of obstructive or inflammatory airways disease, for example asthma, is in the range of from about 50 to about 500 mg per day, in particular from about 100-300 mg per day, conveniently administered in divided doses 2 to 4×/day or in sustained release form. Unit dosage forms for oral administration thus suitably comprise from about 12 to about 500, in particular from about 25 to about 150 or 300 mg compound or ester of the invention, together with a pharmaceutically acceptable diluent or carrier therefor.

The compounds and esters of the invention may be administered in similar manner to known standards, e.g. theophylline, for use in the recited indications, e.g. orally in oral unit dosage form, e.g. in the form of tablets or capsules. They exhibit a low degree of side effects such as psychostimulation as compared with other clinically employed xanthine bronchodilator drugsubstances, for example theophylline or aminophylline.

In accordance with the foregoing the present invention also provides: a pharmaceutical composition comprising a compound of formula I as hereinbefore defined or a physiologically-hydrolysable and -acceptable ester thereof, together with a pharmaceutically acceptable diluent carrier therefor.

## Claims

**Claims for the following Contracting States : BE, CH, DE, FR, GB, IT, LI, LU, NL, SE**

**1.** A compound of formula I

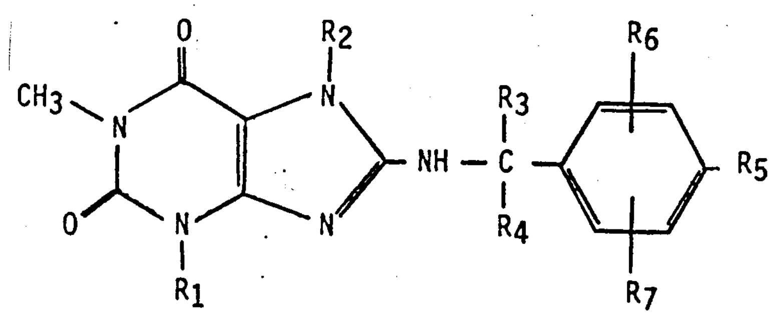

(I)

wherein:

$R_1$ is $C_{1-4}$alkyl, $C_{3-4}$alkenyl or ($C_{3-5}$cycloalkyl)-methyl,

$R_2$ is $C_{1-4}$alkyl, [hydroxy- or ($C_{1-4}$alkoxy)-substituted $C_{1-3}$alkyl]-methyl, ($C_{3-5}$cycloalkyl)-methyl or a group of formula A

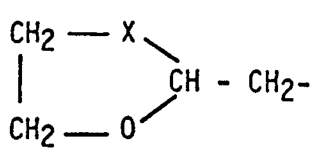

(A)

in which X is $-CH_2-$ or -O-,

$R_3$ and $R_4$ are each hydrogen, hydroxymethyl, methoxymethyl or N,N-dimethylcarbamoyloxymethyl,

| | |
|---|---|
| $R_5$ | is hydroxy or methoxy, |
| $R_6$ | is hydrogen, hydroxy, methoxy or halogen; and |
| $R_7$ | is in the 2- or 3-position and is hydroxy, methoxy or halogen, or together with $R_5$ is 3,4-methylenedioxy, or together with $R_6$ is 2,3-methylenedioxy, |

or physiologically- -hydrolysable and -acceptable ester thereof.

**2.** A compound according to claim 1 wherein

| | |
|---|---|
| $R_1$ | is $C_{1-4}$alkyl, $C_{3-4}$alkenyl or cyclopropylmethyl, |
| $R_2$ | is $C_{1-4}$alkyl, [hydroxy- or ($C_{1-4}$alkoxy)-substituted $C_{1-3}$alkyl]-methyl or a group of formula A as defined in claim 1, |
| $-C(R_3)R_4-$ | is $-CH_2-$, $-CH(CH_2OH)-$, $-CH(CH_2OCH_3)-$, $-C(CH_2OH)_2-$ or $-C(CH_2OCH_3)_2-$, and |
| $R_5$, $R_6$ and $R_7$ | have the meanings given in claim 1, or physiologically-hydrolysable and -acceptable ester thereof. |

**3.** A compound or ester according to claim 2 wherein
$R_3$ is hydrogen, $R_5$ is $CH_3O-$, $R_6$ is hydrogen and $R_7$ is $CH_3O-$ in the 3-position and

a) $R_1$ is $CH_3-$, $R_4$ is hydrogen and $R_7$ is $CH_3-$, $CH_3O(CH_2)_2-$, $HO-(CH_2)_2-$,

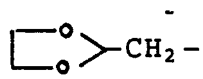

or $CH_3-CO-O-(CH_2)_2-$; or

b) $R_1$ is $CH_2=CH-CH_2-$, $R_4$ is hydrogen and $R_2$ is $CH_3O-(CH_2)_2-$, $HO-(CH_2)_2-$ or

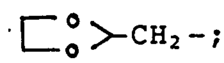

or

c) $R_1$ is

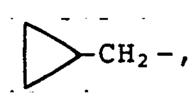

$R_4$ is hydrogen and $R_2$ is $CH_3O-(CH_2)_2-$ or

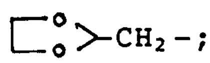

or

d) $R_1$ is $CH_3-$, $R_4$ is $HO-CH_2-$ and $R_2$ is $CH_3-$, $HO-(CH_2)_2-$or

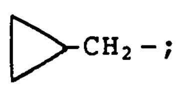

or

e) $R_1$ is $CH_3-$, $R_4$ is $CH_3O-CH_2-$ and $R_2$ is $HO-(CH_2)_2-$; or

f) $R_1$ is $CH_3-$, $R_4$ is $CH_3-CO-O-CH_2-$ or $(CH_3)_2N-CO-O-CH_2-$and $R_2$ is $CH_3O-(CH_2)_2-$; or

g) $R_1$ is $CH_2=CH-CH_2-$, $R_2$ is

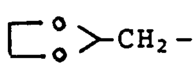

and $R_4$ is $CH_3-CO-O-CH_2-$ or $HO-CH_2-$; or wherein

h) $R_1$ is $CH_3-$, $R_2$ is $CH_3O-(CH_2)_2-$, $R_3$ is hydrogen, $R_4$ is hydrogen, $R_5$ is $CH_3O-$, $R_6$ is hydrogen and $R_7$ is $CH_3O$-in the 2-position.

**4.** A compound according to claim 2 wherein
$R_1$ is $CH_3$-, $R_2$ is $CH_3O$-$(CH_2)_2$-, $R_3$ is hydrogen,
$R_4$ is HO-$CH_2$-, $R_5$ is $CH_3O$-, $R_6$ is hydrogen and
$R_7$ is $CH_3O$- in the 3-position.

**5.** A compound according to claim 2 wherein $R_4$ is $CH_3O$-$CH_2$-and $R_1$, $R_2$, $R_3$, $R_5$, $R_6$ and $R_7$ have the meanings given in claim 4.

**6.** A compound according to any one of claims 1 to 5 wherein -C(R)$_3$$R_4$- is (R) -CH($CH_2OH$)-, (R) -CH-($CH_2OCH_3$)- or (R) -CH[$CH_2$-O-CO-N$(CH_3)_2$]- or pysiologically-hydrolysable and -acceptable ester thereof.

**7.** A compound which is the R-isomer of a compound according to claim 4 or 5.

**8.** A pharmaceutical composition comprising a compound or ester as claimed in any one of claims 1 to 7 together with a pharmaceutically acceptable diluent or carrier therefor.

**9.** A compound or ester as claimed in any one of claims 1 to 7 for use as a pharmaceutical.

**10.** A compound or ester as claimed in any one of claims 1 to 7 for use in the treatment of obstructive or inflammatory airways disease or for the suppression of eosinophil accumulation and/or activation.

**Claims for the following Contracting States : AT, ES, GR**

**1.** Process for the production of a compound of formula I

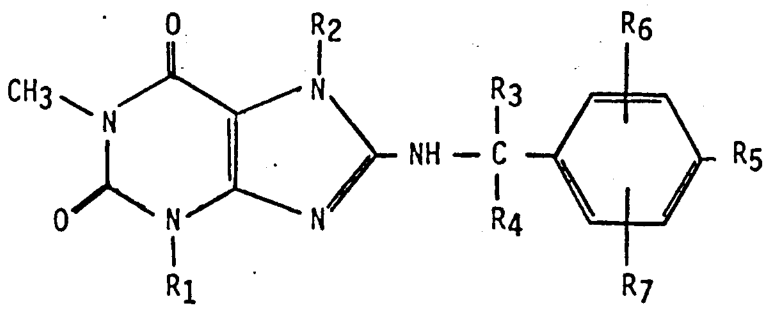

(I)

wherein
$R_1$ is $C_{1-4}$alkyl, $C_{3-4}$alkenyl or ($C_{3-5}$cycloalkyl)-methyl,
$R_2$ is $C_{1-4}$alkyl, [hydroxy- or ($C_{1-4}$alkoxy)-substituted $C_{1-3}$alkyl]-methyl, ($C_{3-5}$cycloalkyl)-methyl or a group of formula A.

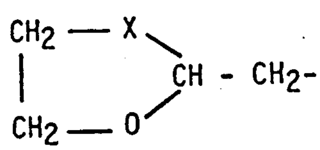

(A)

in which X is -$CH_2$- or -O-,
$R_3$ and $R_4$ are each hydrogen, hydroxymethyl, methoxymethyl or N,N-dimethylcarbamoyloxymethyl,
$R_5$ is hydroxy or methoxy,
$R_6$ is hydrogen, hydroxy, methoxy or halogen and
$R_7$ is in the 2- or 3-position and is hydroxy, methoxy or halogen, or together with $R_5$ is 3,4-methylendioxy, or together with $R_6$ is 2,3-methylenedioxy,
or physiologically-hydrolysable and -acceptable ester thereof,
which process comprises:

a) for the production of a compound of formula I having a free hydroxy group, deprotecting a hydroxy-protected derivative thereof;

b) for the production of a compound of formula I, reacting a compound of formula II

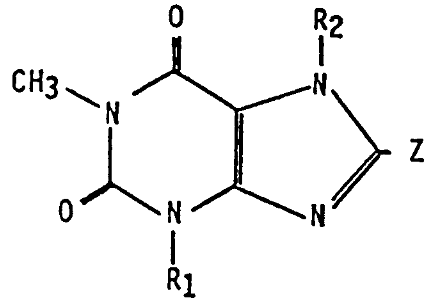

(II)

wherein $R_1$ and $R_2$ have the meanings given for formula I and Z is a leaving group, with a compound of formula III

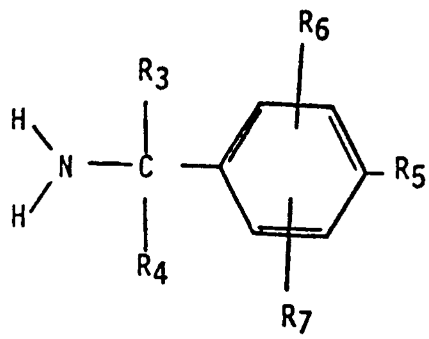

(III)

wherein $R_3$ to $R_7$ have the meanings given for formula I, whereby any hydroxy group present in the compound of formula II or III may be in free or protected form and, when required, carrying out process step a); or

c) for the production of a physiologically-hydrolysable and -acceptable ester of a compound of formula I, esterifying a compound of formula I having a free hydroxy group, any hydroxy group present which is not to be esterified being, when required, in protected form, employing an appropriate acid or derivative thereof and, when required, carrying out process step a); and, if desired,

d) separating optically active isomers from any mixture of such isomers obtained in accordance with steps a), b) or c).

**2.** Process according to claim 1 wherein, in formula I,

| | |
|---|---|
| $R_1$ is | $C_{1-4}$alkyl, $C_{3-4}$alkenyl or cyclopropylmethyl, |
| $R_2$ is | $C_{1-4}$alkyl, [hydroxy- or ($C_{1-4}$alkoxy)-substituted $C_{1-3}$alkyl]-methyl or a group of formula A as defined in claim 1, |
| $-C(R_3)R_4-$ is | $-CH_2-$, $-CH(CH_2OH)-$, $-CH(CH_2OCH_3)-$, $-C(CH_2OH)_2-$ or $-C(CH_2OCH_3)_2-$, and |
| $R_5$, $R_6$ and $R_7$ | have the meanings given in claim 1, or physiologically-hydrolysable and -acceptable ester thereof. |

**3.** Process according to claim 2 wherein, in formula I, $R_3$ is hydrogen, $R_5$ is $CH_3O-$, $R_6$ is hydrogen and $R_7$ is $CH_3O$-in the 3-position and

a) $R_1$ is $CH_3-$, $R_4$ is hydrogen and $R_2$ is $CH_3-$, $CH_3O(CH_2)_2-$, $HO-(CH_2)_2-$,

, 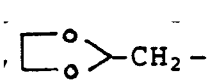

or $CH_3$-CO-O-$(CH_2)_2$-; or
b) $R_1$ is $CH_2$ =CH-$CH_2$-, $R_4$ is hydrogen and $R_2$ is $CH_3O$-$(CH_2)_2$-, HO-$(CH_2)_2$- or

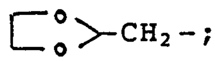

or
c) $R_1$ is

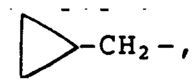

$R_4$ is hydrogen and $R_2$ is $CH_3O$-$(CH_2)_2$- or

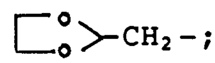

or
d) $R_1$ is $CH_3$-, $R_4$ is HO-$CH_2$- and $R_2$ is $CH_3$-, HO-$(CH_2)_2$-or

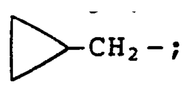

or
e) $R_1$ is $CH_3$-, $R_4$ is $CH_3O$-$CH_2$- and $R_2$ is HO-$(CH_2)_2$-; or
f) $R_1$ is $CH_3$-, $R_4$ is $CH_3$-CO-O-$CH_2$- or $(CH_3)_2$N-CO-O-$CH_2$-and $R_2$ is $CH_3O$-$(CH_2)_2$-; or
g) $R_1$ is $CH_2$ =CH-$CH_2$-, $R_2$ is

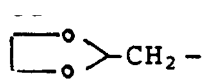

and $R_4$ is $CH_3$-CO-O-$CH_2$- or HO-$CH_2$-; or wherein
h) $R_1$ is $CH_3$-, $R_2$ is $CH_3O$-$(CH_2)_2$-, $R_3$ is hydrogen, $R_4$ is hydrogen, $R_5$ is $CH_3O$-, $R_6$ is hydrogen and $R_7$ is $CH_3O$-in the 2-position.

**4.** Process according to claim 2 wherein, in formula I, $R_1$ is $CH_3$-, $R_2$ is $CH_3O$-$(CH_2)_2$-, $R_3$ is hydrogen, $R_4$ is HO-$CH_2$-, $R_5$ is $CH_3O$-, $R_6$ is hydrogen and $R_7$ is $CH_3O$- in the 3-position.

**5.** Process according to claim 2 wherein, in formula I, $R_4$ is $CH_3O$-$CH_2$- and $R_1$, $R_2$, $R_3$, $R_5$, $R_6$ and $R_7$ have the meanings given in claim 4.

**6.** Process according to any one of claims 1 to 5 wherein, in formula I, -C(R)$_3$$R_4$- is (R) -CH($CH_2$OH)-, (R) -CH($CH_2OCH_3$)- or (R) -CH[$CH_2$-O-CO-N$(CH_3)_2$].

**7.** Process according to claim 4 or 5, wherein the compound of formula I is in the form of its R-isomer.

**8.** A compound or ester wherever prepared by a process as claimed in any one of claims 1 to 7.

**9.** A pharmaceutical composition comprising a compound of formula I as illustrated in claim 1, wherein $R_1$ to $R_7$ have the meanings given in any one of claims 1 to 7 or a physiologically-hydrolysable and -acceptable ester thereof or a compound or ester as claimed in claim 8, together with a pharmaceutically acceptable diluent or carrier therefor.

**10.** A compound of formula I as illustrated in claim 1, wherein $R_1$ to $R_7$ have the meanings given in any one of claims 1 to 7 or a physiologically-hydrolysable and -acceptable ester thereof or a compound or

ester as claimed in claim 8, for use as a pharmaceutical.

**Revendications**

**Revendications pour les Etats contractants suivants : BE, CH, DE, FR, GB, IT, LI, LU, NL, SE**

**1.** Un composé de formule I

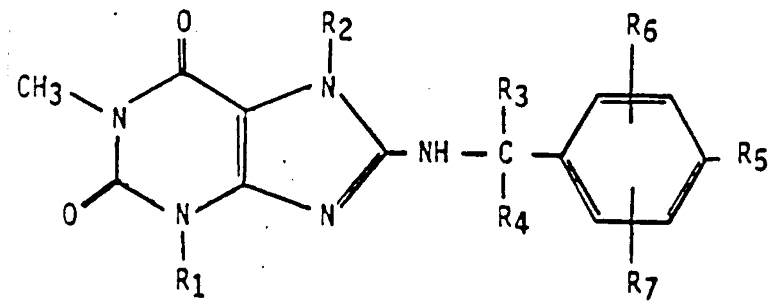

(I)

dans laquelle

$R_1$ signifie un groupe alkyle en $C_1$-$C_4$, alcényle en $C_3$-$C_4$ ou (cycloalkyl en $C_3$-$C_5$)-méthyle,

$R_2$ signifie un groupe alkyle en $C_1$-$C_4$, [hydroxy-alkyl en $C_1$-$C_3$ ou (alcoxy en $C_1$-$C_4$ )-alkyl en $C_1$-$C_3$]-méthyle, (cycloalkyl en $C_3$-$C_5$)-méthyle ou un groupe de formule A

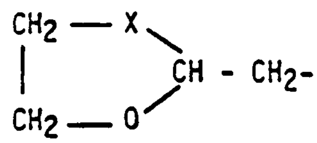

(A)

dans laquelle X signifie $-CH_2-$ ou -O-,

$R_3$ et $R_4$ signifient chacun l'hydrogène ou un groupe hydroxyméthyle, méthoxyméthyle ou N,N-diméthylcarbamoyloxyméthyle,

$R_5$ signifie un groupe hydroxy ou méthoxy,

$R_6$ signifie l'hydrogène, un groupe hydroxy, méthoxy ou un halogène et

$R_7$ est en position 2 ou 3 et signifie un groupe hydroxy, méthoxy ou un halogène, ou ensemble avec $R_5$ signifie un groupe 3,4-méthylènedioxy, ou ensemble avec $R_6$ signifie un groupe 2,3-méthylènedioxy,

ou un ester physiologiquement hydrolysable et physiologiquement acceptable de ce compose.

**2.** Un composé selon la revendication 1, dans lequel

$R_1$ signifie un groupe alkyle en $C_1$-$C_4$, alcényle en $C_3$-$C_4$ ou cyclopropylméthyle,

$R_2$ signifie un groupe alkyle en $C_1$-$C_4$, [hydroxy-alkyl en $C_1$-$C_3$ ou (alcoxy en $C_1$-$C_4$)-alkyl en $C_1$-$C_3$]-méthyle ou un groupe de formule A tel que défini à la revendication 1,

$-C(R_3)R_4-$ signifie $-CH_2-$, $-CH(CH_2OH)-$, $-CH(CH_2OCH_3)-$, $-C(CH_2OH)_2-$ ou $-C(CH_2OCH_3)_2-$, et

$R_5$, $R_6$ et $R_7$ ont les significations données à la revendication 1,

ou un ester physiologiquement hydrolysable et physiologiquement acceptable de ce composé.

**3.** Un composé ou un ester selon la revendication 2, dans lequel

$R_3$ signifie l'hydrogène, $R_5$ signifie $CH_3O-$, $R_6$ signifie l'hydrogène et R, signifie $CH_3$-O- en position 3 et

a) $R_1$ signifie $CH_3-$, $R_4$ signifie l'hydrogène et $R_2$ signifie $CH_3-$, $CH_3O(CH_2)_2-$, $HO-(CH_2)_2-$,

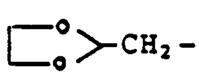

ou $CH_3-CO-O-(CH_2)_2-$, ou bien

b) $R_1$ signifie $CH_2=CH\text{-}CH_2\text{-}$, $R_4$ signifie l'hydrogène et $R_2$ signifie $CH_3O\text{-}(CH_2)_2\text{-}$, $HO\text{-}(CH_2)_2\text{-}$ ou

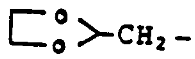 ,

ou bien

c) $R_1$ signifie

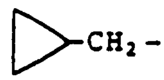 ,

$R_4$ signifie l'hydrogène et $R_2$ signifie $CH_3O\text{-}(CH_2)_2\text{-}$ ou

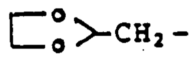 ,

ou bien

d) $R_1$ signifie $CH_3\text{-}$, $R_4$ signifie $HO\text{-}CH_2\text{-}$ et $R_2$ signifie $CH_3\text{-}$, $HO\text{-}(CH_2)_2\text{-}$ ou

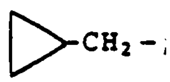 ,

ou bien

e) $R_1$ signifie $CH_3\text{-}$, $R_4$ signifie $CH_3O\text{-}CH_2\text{-}$ et $R_2$signifie $HO\text{-}(CH_2)_2\text{-}$, ou bien

f) $R_1$ signifie $CH_3\text{-}$, $R_4$ signifie $CH_3\text{-}CO\text{-}O\text{-}CH_2\text{-}$ ou $(CH_3)_2N\text{-}CO\text{-}O\text{-}CH_2\text{-}$ et $R_2$ signifie $CH_3O\text{-}(CH_2)_2\text{-}$ ,ou bien

g) $R_1$ signifie $CH_2=CH\text{-}CH_2\text{-}$, $R_2$ signifie

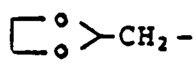

et $R_4$ signifie $CH_3\text{-}CO\text{-}O\text{-}CH_2\text{-}$ou $HO\text{-}CH_2\text{-}$, ou dans lequel

h) $R_1$ signifie $CH_3\text{-}$, $R_2$ signifie $CH_3O\text{-}(CH_2)_2\text{-}$, $R_3$ signifie l'hydrogène, $R_4$ signifie l'hydrogène, $R_5$ signifie $CH_3O\text{-}$, $R_6$ signifie l'hydrogène et $R_7$ signifie $CH_3O\text{-}$ en position 2.

**4.** Un composé selon la revendication 2, dans lequel
$R_1$ signifie $CH_3\text{-}$, $R_2$ signifie $CH_3O\text{-}(CH_2)_2\text{-}$, $R_3$ signifie l'hydrogène,
$R_4$ signifie $HO\text{-}CH_2\text{-}$, $R_5$ signifie $CH_3O\text{-}$, $R_6$ signifie l'hydrogène et
$R_7$ signifie $CH_3O\text{-}$ en position 3.

**5.** Un composé selon la revendication 2, dans lequel $R_4$ signifie $CH_3O\text{-}CH_2\text{-}$ et $R_1$, $R_2$, $R_3$, $R_5$, $R_6$ et $R_7$ ont les significations données à la revendication 4.

**6.** Un composé selon l'une quelconque des revendications 1 à 5, dans lequel $\text{-}C(R)_3R_4\text{-}$ signifie (R) $\text{-}CH(CH_2OH)\text{-}$, (R) $\text{-}CH(CH_2OCH_3)\text{-}$ ou (R)$\text{-}CH[CH_2\text{-}O\text{-}CO\text{-}N(CH_3)_2]\text{-}$, ou un ester physiologiquement hydrolysable et physiologiquement acceptable de ce composé.

**7.** Un composé qui est l'isomère R d'un composé selon la revendication 4 ou 5.

**8.** Une composition pharmaceutique, comprenant un composé ou un ester tel que spécifié à l'une quelconque des revendications 1 à 7, ensemble avec un diluant ou véhicule pharmaceutiquement acceptable.

**9.** Un composé ou un ester tel que spécifié à l'une quelconque des revendications 1 à 7, pour l'utilisation comme médicament.

**10.** Un composé ou un ester tel que spécifié à l'une quelconque des revendications 1 à 7, pour l'utilisation dans le traitement des maladies obstructives ou inflammatoires des voies respiratoires ou pour la suppression de l'accumulation et/ou de l'activation des éosinophiles.

**Revendications pour les Etats contractants suivants : AT, ES, GR**

**1.** Un procédé de préparation d'un composé de formule I

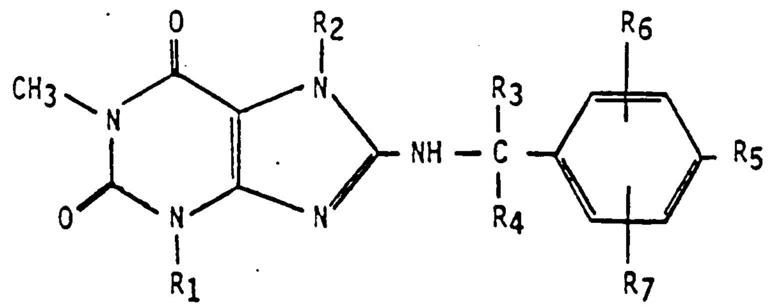

(I)

dans laquelle

$R_1$ signifie un groupe alkyle en $C_1$-$C_4$, alcényle en $C_3$-$C_4$ ou (cycloalkyl en $C_3$-$C_5$)-méthyle,

$R_2$ signifie un groupe alkyle en $C_1$-$C_4$, [hydroxy-alkyl en $C_1$-$C_3$ ou (alcoxy en $C_1$-$C_4$)-alkyl en $C_1$-$C_3$]-méthyle, (cycloalkyl en $C_3$-$C_5$)-méthyle ou un groupe de formule A

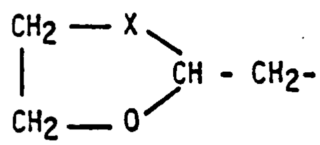

**(A)**

dans laquelle X signifie $-CH_2-$ ou -O-,

$R_3$ et $R_4$ signifient chacun l'hydrogène ou un groupe hydroxyméthyle, méthoxyméthyle ou N,N-diméthylcarbamoyloxyméthyle,

$R_5$ signifie un groupe hydroxy ou méthoxy,

$R_6$ signifie l'hydrogène, un groupe hydroxy, méthoxy ou un halogène et

$R_7$ est en position 2 ou 3 et signifie un groupe hydroxy, méthoxy ou un halogène, ou ensemble avec $R_5$ signifie un groupe 3,4-méthylènedioxy, ou ensemble avec $R_6$ signifie un groupe 2,3-méthylènedioxy,

ou d'un ester physiologiquement hydrolysable et physiologiquement acceptable de ce composé, lequel procédé comprend:

a) pour la préparation d'un composé de formule I ayant un groupe hydroxy libre, la déprotection d'un dérivé de ce composé à groupe hydroxy protégé,

b) pour la préparation d'un composé de formule T, la réaction d'un composé de formule II

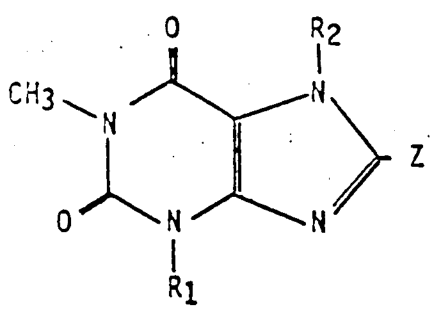

(II)

dans laquelle $R_1$ et $R_2$ ont les significations données pour la formule I et Z signifie un groupe éliminable, avec un composé de formule III

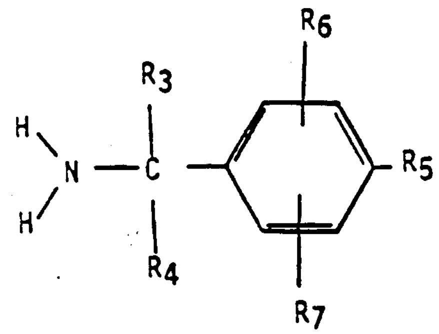

(III)

dans laquelle $R_3$ à $R_7$ ont les significations données pour la formule I, tout groupe hydroxy présent dans le composé de formule II ou III pouvant être sous forme libre ou protégée et, si nécessaire, la mise en oeuvre de l'étape a) du procédé, ou

c) pour la préparation d'un ester physiologiquement hydrolysable et physiologiquement acceptable d'un composé de formule I, l'estérification d'un compose de formule I ayant un groupe hydroxy libre, tout groupe hydroxy présent qui ne doit pas être estérifié étant, si nécessaire, sous forme protégée, en utilisant un acide approprié ou l'un de ses dérivés et, si nécessaire, la mise en oeuvre de l'étape a) du procédé, et, si on le désire,

d) la séparation des isomères optiquement actifs de tout mélange de tels isomères obtenu selon l'étape a), b) ou c).

**2.** Un procédé selon la revendication 1, dans lequel, dans la formule I,

| | |
|---|---|
| $R_1$ | signifie un groupe alkyle en $C_1$-$C_4$, alcényle en $C_3$-$C_4$ ou cyclopropylméthyle, |
| R | $_2$ signifie un groupe alkyle en $C_1$-$C_4$, [hydroxy-alkyl en $C_1$-$C_3$ ou (alcoxy en $C_1$-$C_4$)-alkyl en $C_1$-$C_3$]-méthyle ou un groupe de formule A tel que défini à la revendication 1, |
| $-C(R_3)R_4-$ | signifie $-CH_2-$, $-CH(CH_2OH)-$, $-CH(CH_2OCH_3)-$, $-C(CH_2OH)_2-$ ou $-C(CH_2OCH_3)_2-$, et |
| $R_5$, $R_6$ et $R_7$ | ont les significations données à la revendication 1, |

ou un ester physiologiquement hydrolysable et physiologiquement acceptable de ce composé.

**3.** Un procédé selon la revendication 2, dans lequel, dans la formule I,

$R_3$ signifie l'hydrogène, $R_5$ signifie $CH_3O-$, $R_6$ signifie l'hydrogène et $R_7$ signifie $CH_3-O$ en position 3 et

a) $R_1$ signifie $CH_3-$, $R_4$ signifie l'hydrogène et $R_2$ signifie $CH_3-$, $CH_3O(CH_2)_2-$, $HO-(CH_2)_2-$,

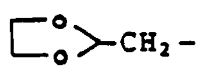

ou $CH_3-CO-O-(CH_2)_2-$, ou bien

b) $R_1$ signifie $CH_2=CH-CH_2-$, $R_4$ signifie l'hydrogène et $R_2$ signifie $CH_3O-(CH_2)_2-$, $HO-(CH_2)_2-$ ou

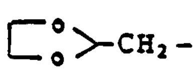 ,

ou bien

c) $R_1$ signifie

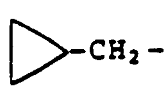 ,

$R_4$ signifie l'hydrogène et $R_2$ signifie $CH_3O-(CH_2)_2-$ou

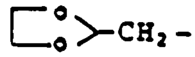 ,

ou bien
d) $R_1$ signifie $CH_3$-, $R_4$ signifie HO-$CH_2$- et $R_2$ signifie $CH_3$-, HO-$(CH_2)_2$- ou

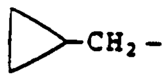 ,

ou bien
e) $R_1$ signifie $CH_3$-, $R_4$ signifie $CH_3O$-$CH_2$- et $R_2$ signifie HO-$(CH_2)_2$-, ou bien
f) $R_1$ signifie $CH_3$-, $R_4$ signifie $CH_3$-CO-O-$CH_2$- ou $(CH_3)_2N$-CO-O-$CH_2$- et $R_2$ signifie $CH_3O$-$(CH_2)_2$-, ou bien
g) $R_1$ signifie $CH_2$=CH-$CH_2$-, $R_2$ signifie

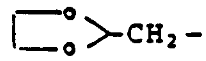

et $R_4$ signifie $CH_3$-CO-O-$CH_2$- ou HO-$CH_2$-, ou dans lequel
h) $R_1$ signifie $CH_3$-, $R_2$ signifie $CH_3O$-$(CH_2)_2$-, $R_3$ signifie l'hydrogène, $R_4$ signifie l'hydrogène, $R_5$ signifie $CH_3O$-, $R_6$ signifie l'hydrogène et $R_7$ signifie $CH_3O$- en position 2.

**4.** Un procédé selon la revendication 2, dans lequel, dans la formule I, $R_1$ signifie $CH_3$-, $R_2$ signifie $CH_3O$-$(CH_2)_2$-, $R_3$ signifie l'hydrogène, $R_4$ signifie HO-$CH_2$-, $R_5$ signifie $CH_3O$-, $R_6$ signifie l'hydrogène et $R_7$ signifie $CH_3O$- en position 3.

**5.** Un procédé selon la revendication 2, dans lequel, dans la formule I, $R_4$ signifie $CH_3O$-$CH_2$-et $R_1$, $R_2$, $R_3$, $R_5$, $R_6$ et $R_7$ ont les significations données à la revendication 4.

**6.** Un procédé selon l'une quelconque des revendications 1 à 5, dans lequel, dans la formule I, -C(R)$_3$R$_4$- signifie (R) -CH($CH_2OH$)-, (R) -CH($CH_2OCH_3$)-ou (R) -CH[$CH_2$-O-CO-N$(CH_3)_2$]-.

**7.** Un procédé selon la revendication 4 ou 5, dans lequel le composé de formule I est sous forme de son isomère R.

**8.** Un composé ou un ester lorsqu'il est préparé selon un procédé tel que spécifié à l'une quelconque des revendications 1 à 7.

**9.** Une composition pharmaceutique, comprenant un composé de formule I telle qu'illustrée à la revendication 1, où $R_1$ à $R_7$ ont les significations données à l'une quelconque des revendications 1 à 7 ou un ester physiologiquement hydrolysable et physiologiquement acceptable de ce composé ou un composé ou un ester tel que spécifié à la revendication 8, ensemble avec un diluant ou un véhicule pharmaceutiquement acceptable.

**10.** Un composé de formule I telle qu'illustrée à la revendication 1, où $R_1$ à $R_7$ ont les significations données à l'une quelconque des revendications 1 à 7 ou un ester physiologiquement hydrolysable et physiologiquement acceptable de ce composé ou un composé ou un ester tel que spécifié à la revendication 8, pour l'utilisation comme médicament.

**Patentansprüche**
**Patentansprüche für folgende Vertragsstaaten : BE, CH, DE, FR, GB, IT, LI, LU, NL, SE**

**1.** Verbindung der Formel I

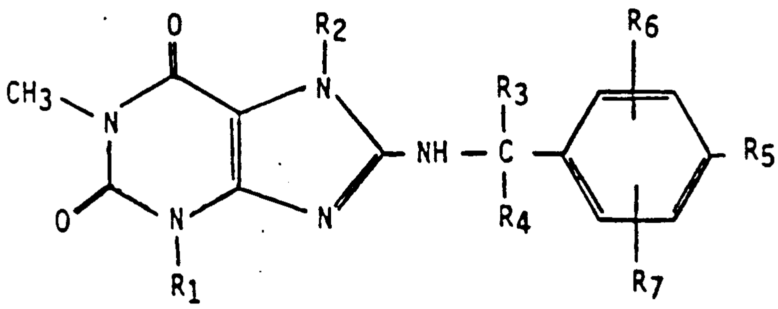

(I)

worin

$R_1$ ein $C_1$-$C_4$-Alkyl-, $C_3$-$C_4$--Alkenyl- oder ($C_3$-$C_5$-Cycloalkyl) methylrest ist,

$R_2$ ein $C_1$-$C_4$-Alkyl-, [Hydroxy- oder ($C_1$-$C_4$-Alkoxy)-substituierter$C_1$-$C_3$-Alkyl]methyl-, ($C_3$-$C_5$-Cycloalkyl)methylrest oder eine Gruppe der Formel A

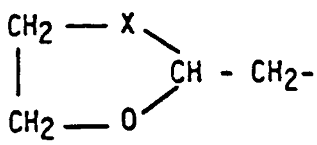

(A)

ist, worin X $-CH_2$- oder -O- ist,

$R_3$ und $R_4$ jeweils Wasserstoff, ein Hydroxymethyl-, Methoxymethyl- oder N,N-Dimethylcarbamoyloxymethylrest ist,

$R_5$ ein Hydroxy- oder Methoxyrest ist,

$R_6$ Wasserstoff, ein Hydroxyrest, Methoxyrest oder Halogen ist und

$R_7$ sich in 2- oder 3-Stellung befindet und ein Hydroxy-, Methoxyrest oder Halogen ist oder zusammen mit $R_5$ ein 3,4-Methylendioxyrest ist oder zusammen mit $R_6$ ein 2,3-Methylendioxyrest ist,

oder physiologisch hydrolysierbare und annehmbare Ester davon.

**2.** Verbindung nach Anspruch 1, worin

$R_1$ ein $C_1$-$C_4$-Alkyl-, $C_3$-$C_4$-Alkenyl- oder Cyclopropylmethylrest ist,

$R_2$ ein $C_1$-$C_4$-Alkyl-, [Hydroxy- oder ($C_1$-$C_4$-Alkoxy)-substituierter$C_1$-$C_3$-Alkyl]-methylrest oder eine Gruppe der Formel A, wie in Anspruch 1 definiert, ist,

$-C(R_3)R_4$- $-CH_2$-, $-CH(CH_2OH)$-, $-CH(CH_2OCH_3)$-, $-C(CH_2OH)_2$- oder $C(CH_2OCH_3)_2$- ist und

$R_5$, $R_6$ und $R_7$ die in Anspruch 1 angegebenen Bedeutungen haben, oder physiologisch hydrolysierbare und annehmbare Ester davon.

**3.** Verbindung oder Ester nach Anspruch 2, worin $R_3$ Wasserstoff ist, $R_5$ $CH_3O$- ist, $R_6$ Wasserstoff ist und $R_7$ $CH_3O$- in 3-Position ist und

a) $R_1$ $CH_3$- ist, $R_4$ Wasserstoff ist und $R_2$ $CH_3$-, $CH_3O(CH_2)_2$-, $HO-(CH_2)_2$-,

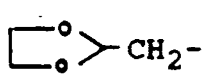

oder $CH_3-CO-O-(CH_2)_2$- ist; oder

b) $R_1$ $CH_2=CH-CH_2$- ist, $R_6$ Wasserstoff ist und $R_2$ $CH_3O-(CH_2)_2$-, $HO-(CH_2)_2$- oder

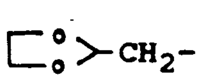

ist; oder

c) $R_1$

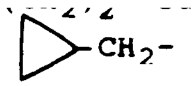

ist, $R_4$ Wasserstoff ist und $R_2$ $CH_3O-(CH_2)_2$-oder

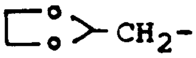

ist; oder

d) $R_1$ $CH_2$- ist, $R_4$ HO-$CH_2$- ist und $R_2$ $CH_3$-, HO-$(CH_2)_2$- oder

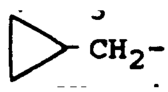

ist; oder

e) $R_1$ $CH_3$- ist, $R_4$ $CH_3O-CH_2$- ist und $R_2$ HO-$(CH_2)_2$- ist; oder

f) $R_1$ $CH_3$- ist, $R_4$ $CH_3$-CO-O-$CH_2$- oder $(CH_3)_2$N-CO-O-$CH_2$- ist und $R_2$ $CH_3O-(CH_2)_2$- ist; oder

g) $R_1$ $CH_2=CH-CH_2$- ist, $R_2$

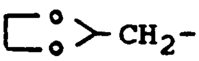

ist und $R_4$ $CH_3$-CO-O-$CH_2$-oder HO-$CH_2$- ist; oder worin

h) $R_1$ $CH_3$- ist, $R_2$ $CH_3O-(CH_2)_2$- ist, $R_3$ Wasserstoff ist, $R_4$ Wasserstoff ist, $R_5$ $CH_3O$- ist, $R_6$ Wasserstoff ist und $R_7$ $CH_3O$- in 2-Stellung ist.

**4.** Verbindung nach Anspruch 2, worin $R_1$ $CH_3$- ist, $R_2$ $CH_3O-(CH_2)_2$- ist, $R_3$ Wasserstoff ist, $R_4$ HO-$CH_2$-ist, $R_5$ $CH_3O$-ist, $R_6$ Wasserstoff ist und $R_7$ $CH_3O$- in 3-Stellung ist.

**5.** Verbindung nach Anspruch 2, worin $R_4$ $CH_3O-CH_2$- ist und $R_1$, $R_2$, $R_3$, $R_5$, $R_6$ und $R_7$ die in Anspruch 4 angegebenen Bedeutungen haben.

**6.** Verbindung nach einem der Ansprüche 1 bis 5, worin -C($R_3$)$R_4$-(R) -CH($CH_2OH$)-, (R) -CH($CH_2OCH_3$)-oder (R) -CH[$CH_2$-O-CO-N$(CH_3)_2$]- oder ein physiologisch hydrolysierbarer oder annehmbarer Ester davon ist.

**7.** Verbindung, die das R-Isomer einer Verbindung gemäß Anspruch 4 oder 5 ist.

**8.** Pharmazeutische Zusammensetzung enthaltend eine Verbindung oder einen Ester nach einem der Ansprüche 1 bis 7 zusammen mit einem pharmazeutisch annehmbaren Verdünnungsmittel oder Träger.

**9.** Verbindung oder Ester nach einem der Ansprüche 1 bis 7 zur Verwendung als Pharmazeutikum.

**10.** Verbindung oder Ester nach einem der Ansprüche 1 bis 7 zur Verwendung zur Behandlung einer obstruktiven oder inflammatorischen Luftwegserkrankung oder für die Unterdrückung der Ansammlung und/oder Aktivierung von Eosinophilen.

**Patentansprüche für folgende Vertragsstaaten : AT, ES, GR**

**1.** Verfahren zur Herstellung einer Verbindung der Formel I

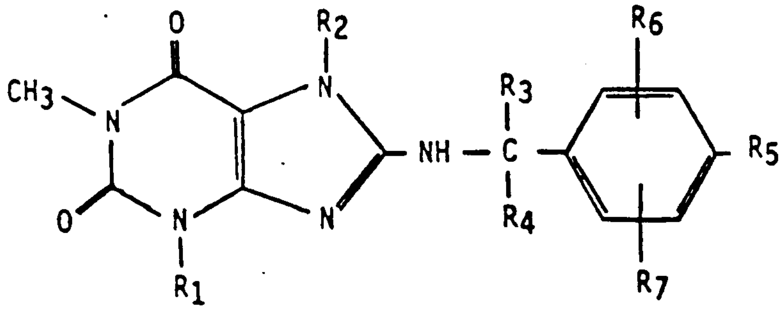

(I)

worin

$R_1$ ein $C_1$-$C_4$-Alkyl-, $C_3$-$C_4$-Alkenyl- oder ($C_3$-$C_5$-Cycloalkyl) methylrest ist,

$R_2$ ein $C_1$-$C_4$-Alkyl-, [Hydroxy- oder ($C_1$-$C_4$-Alkoxy)-substituierter$C_1$-$C_3$-Alkyl]methyl-, ($C_3$-$C_5$-Cycloalkyl)methylrest oder eine Gruppe der Formel A

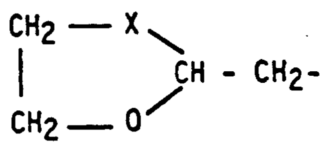

(A)

ist, worin X -$CH_2$- oder -O- ist,

$R_3$ und $R_4$ jeweils Wasserstoff, ein Hydroxymethyl-, Methoxymethyl- oder N,N-Dimethylcarbamoyloxymethylrest ist,

$R_5$ ein Hydroxy- oder Methoxyrest ist,

$R_6$ Wasserstoff, ein Hydroxyrest, Methoxyrest oder Halogen ist und

$R_7$ sich in 2- oder 3-Stellung befindet und ein Hydroxy-, Methoxyrest oder Halogen ist oder zusammen mit $R_5$ ein 3,4-Methylendioxyrest ist oder zusammen mit $R_6$ ein 2,3-Methylendioxyrest ist,

oder eines physiologisch hydrolysierbaren und annehmbaren Esters davon,
wobei dieses Verfahren umfaßt:

a) zur Herstellung einer Verbindung der Formel I mit einer freien Hydroxygruppe, daß man von einem Derivat mit geschützter Hydroxygruppe die Schutzgruppe abspaltet;

b) zur Herstellung einer Verbindung der Formel I eine Verbindung der Formel II

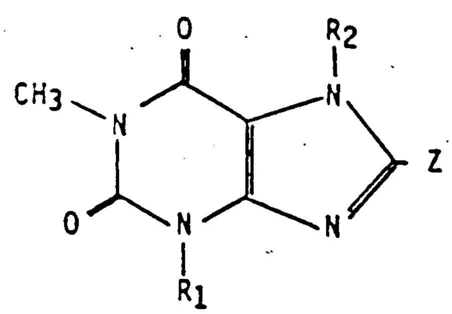

(II)

worin $R_1$ und $R_2$ die für Formel I angegebenen Bedeutungen haben und Z eine Abgangsgruppe ist, mit einer Verbindung der Formel III

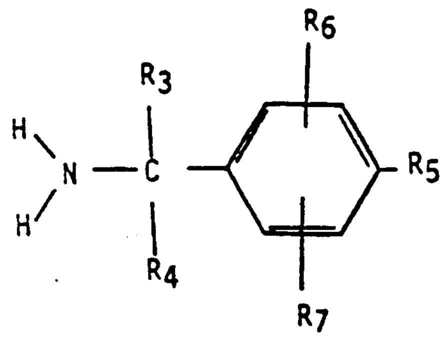

(III)

worin $R_3$ bis $R_7$ die für Formel I angegebenen Bedeutungen haben, wobei jegliche in der Verbindung der Formel II oder III vorhandene Hydroxygruppe in freier Form oder geschützter Form sein kann, umsetzt und falls erforderlich Verfahrensstufe a) durchführt; oder

c) zur Herstellung eines physiologisch hydrolysierbaren und annehmbaren Esters einer Verbindung der Formel I eine Verbindung der Formel I mit einer freien Hydroxygruppe verestert, wobei jede vorhandene Hydroxygruppe, die nicht verestert wird, falls erforderlich, in geschützter Form vorliegen kann, unter Anwendung einer geeigneten Säure oder eines Derivats davon und falls erforderlich Verfahrensstufe a) durchführt; und, falls erwünscht,

d) optisch aktive Isomere aus einer Mischung solcher Isomeren, die gemäß den Stufen a), b) oder c) erhalten wurden, auftrennt.

**2.** Verfahren nach Anspruch 1, worin in Formel I

| | |
|---|---|
| $R_1$ | ein $C_1$-$C_4$-Alkyl-, $C_3$-$C_4$-Alkenyl- oder Cyclopropylmethylrest ist, |
| $R_2$ | ein $C_1$-$C_4$-Alkyl-, [Hydroxy- oder ($C_1$-$C_4$-Alkoxy)-substituierter$C_1$-$C_3$-Alkyl]-methylrest oder eine Gruppe der Formel A, wie in Anspruch 1 definiert, ist, |
| $-C(R_3)R_4-$ | $-CH_2-$, $-CH(CH_2OH)-$, $-CH(CH_2OCH_3)-$, $-C(CH_2OH)_2-$ oder $C(CH_2OCH_3)_2-$ ist und |
| $R_5$, $R_6$ und $R_7$ | die in Anspruch 1 angegebenen Bedeutungen haben, oder physiologisch hydrolysierbare und annehmbare Ester davon. |

**3.** Verfahren nach Anspruch 2, worin in Formel I $R_3$ Wasserstoff ist, $R_5$ $CH_3O$- ist, $R_6$ Wasserstoff ist und $R_7$ $CH_3O$- in 3-Position ist und

a) $R_1$ $CH_3$- ist, $R_4$ Wasserstoff ist und $R_2$ $CH_3$-, $CH_3O(CH_2)_2$-, $HO-(CH_2)_2$-,

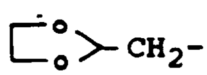

oder $CH_3-CO-O-(CH_2)_2$- ist; oder

b) $R_1$ $CH_2=CH-CH_2$- ist, $R_4$ Wasserstoff ist und $R_2$ $CH_3O-(CH_2)_2$-, $HO-(CH_2)_2$- oder

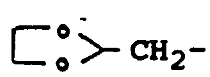

ist; oder

c) $R_1$

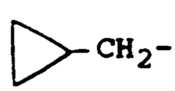

ist, $R_4$ Wasserstoff ist und $R_2$ $CH_3O-(CH_2)_2$-oder

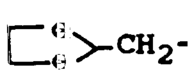

ist; oder

d) $R_1$ $CH_3$- ist, $R_4$ $HO-CH_2$- ist und $R_2$ $CH_3$-, $HO-(CH_2)_2$- oder

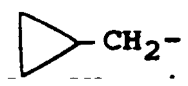

ist; oder

e) $R_1$ $CH_3$- ist, $R_4$ $CH_3O$-$CH_2$- ist und $R_2$ HO-$(CH_2)_2$- ist; oder

f) $R_1$ $CH_3$- ist, $R_4$ $CH_3$-CO-O-$CH_2$- oder $(CH_3)_2$N-CO-O-$CH_2$- ist und $R_2$ $CH_3O$-$(CH_2)_2$- ist; oder

g) $R_1$ $CH_2$ = CH-$CH_2$- ist, $R_2$

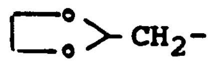

ist und $R_4$ $CH_3$-CO-O-$CH_2$-oder HO-$CH_2$- ist; oder worin

h) $R_1$ $CH_3$- ist, $R_2$ $CH_3O$-$(CH_2)_2$- ist, $R_3$ Wasserstoff ist, $R_4$ Wasserstoff ist, $R_5$ $CH_3O$- ist, $R_6$ Wasserstoff ist und $R_7$ $CH_3O$- in 2-Stellung ist.

**4.** Verfahren nach Anspruch 2, worin in Formel I $R_1$ $CH_3$- ist, $R_2$ $CH_3O$-$(CH_2)_2$- ist, $R_3$ Wasserstoff ist, $R_4$ HO-$CH_2$- ist, $R_5$ $CH_3O$-ist, $R_6$ Wasserstoff ist und $R_7$ $CH_3O$- in 3-Stellung ist.

**5.** Verfahren nach Anspruch 2, worin in Formel I $R_4$ $CH_3O$-$CH_2$-ist und $R_1$, $R_2$, $R_3$, $R_5$, $R_6$ und $R_7$ die in Anspruch 4 angegebenen Bedeutungen haben.

**6.** Verfahren nach einem der Ansprüche 1 bis 5, worin in Formel I -C($R_3$)$R_4$- (R) -CH($CH_2OH$)-, (R) -CH-($CH_2OCH_3$)- oder (R) -CH[$CH_2$-O-CO-N$(CH_3)_2$]- ist.

**7.** Verfahren nach Anspruch 4 oder 5, worin die Verbindung der Formel I in Form des R-Isomers ist.

**8.** Verbindung oder Ester hergestellt nach einem Verfahren nach einem der Ansprüche 1 bis 7.

**9.** Pharmazeutische Zusammensetzung enthaltend eine Verbindung der Formel I wie in Anspruch 1 dargestellt, worin $R_1$ bis $R_7$ die in einem der Ansprüche 1 bis 7 angegebenen Bedeutungen haben, oder einen physiologisch hydrolysierbaren und annehmbaren Ester oder eine Verbindung oder einen Ester nach Anspruch 8 zusammen mit einem pharmazeutisch annehmbaren Verdünnungsmittel oder Träger.

**10.** Verbindung der Formel I, wie in Anspruch 1 dargestellt, worin $R_1$ bis $R_7$ die in einem der Ansprüche 1 bis 7 angegebenen Bedeutungen haben, oder ein physiologisch hydrolysierbarer und annehmbarer Ester davon oder eine Verbindung oder ein Ester nach Anspruch 8 zur Verwendung als Pharmazeutikum.